# EUROPEAN PATENT APPLICATION

(11) **EP 4 008 190 A1**
(43) Date of publication of application: **08.06.2022**
(21) Application number: 20849518.4
(22) Date of filing: 19.06.2020
(51) Int. Cl.: A23J 3/08, C07K 1/14

(54) **METHOD FOR PRODUCING GLYCOMACROPEPTIDE**

(30) Priority: 02.08.2019 JP 2019142768
(71) Applicant: AJINOMOTO CO., INC., Chuo-ku Tokyo 104-8315 (JP)
(72) Inventor: ENDO, Yuta, Kawasaki-shi, Kanagawa 210-8681 (JP); KISHINO, Mitsuhiro, Kawasaki-shi, Kanagawa 210-8681 (JP); KANEKO, Masaya, Kawasaki-shi, Kanagawa 210-8681 (JP)
(74) Representative: Strehl Schübel-Hopf & Partner
(86) International application number: PCT/JP2020/024228
(87) International publication number: WO 2021/024622

(57) **Abstract**

There is provided a method capable of easily producing high-purity glycomacropeptide with a low phenylalanine content on an industrial scale. Specifically, there is provided a method for producing glycomacropeptide, including: (A) bringing a whey protein mixture containing glycomacropeptide into contact with activated carbon; and (B) separating the glycomacropeptide from the activated carbon.

## Description

### TECHNICAL FIELD

The present invention relates to a method for producing glycomacropeptide, and in particular, to a method for easily producing high-purity glycomacropeptide with a low phenylalanine content on an industrial scale.

### BACKGROUND ART

Phenylketonuria is a disorder in which a patient congenitally lacks the function of phenylalanine hydroxylase, which is involved in phenylalanine metabolism, and intake of phenylalanine is restricted in such a patient. Therefore, the development of a protein source that is easy to ingest for the patient with phenylketonuria is required.

Glycomacropeptide is a protein having a molecular weight of about 8,000 (without glycosylation) or a molecular weight of 25,000 to 30,000 (with glycosylation) and containing no phenylalanine residue in its amino acid sequence. Glycomacropeptide is present in whey, a byproduct from the production of dairy products such as cheese. Since glycomacropeptide contains no phenylalanine residues in its amino acid sequence, glycomacropeptide is used as the protein source that is easy to ingest for a patient with phenylketonuria. Since a large amount of whey is secondarily produced in the production of dairy products, glycomacropeptide available from whey is considered to be suitable for industrial production.

However, glycomacropeptide industrially obtained from whey contains a phenylalanine component (e.g., free phenylalanine, and a peptide and a protein containing a phenylalanine residue) as an impurity, and complete removal of the phenylalanine component is difficult. Therefore, the development of a method for producing, from whey, glycomacropeptide in which the phenylalanine component is removed as much as possible is required.

As such a method, there are some methods, based on a hydrophilic interaction, using ion exchange resins (ion exchange chromatography).

Non-patent Literature 1 describes a method for producing glycomacropeptide in which a phenylalanine component has been removed by some cycles of separation using a cation exchange resin and the like.

Non-patent Literature 2 describes a method for producing glycomacropeptide in which a phenylalanine component has been removed by acidic precipitation and separation using an anion exchange resin and the like.

Non-patent Literature 3 describes a method for producing glycomacropeptide in which a phenylalanine component has been removed by separation using an anion exchange resin and the like.

However, these methods require the use of ion exchange resins, and include an elution step using a salt solution (in many cases, containing a high concentration of salt). Therefore, the salt is mixed in a glycomacropeptide eluted solution. Since salts adversely affect the flavor of foods, mixing of the salts is not desired. A procedural step of exchanging the salts by a technique such as dialysis after mixing of the salts is complicated, and is disadvantageous in terms of cost. Therefore, the development of a method that does not require elution step using a salt solution is desired. Furthermore, the methods using ion exchange resins are disadvantageous in terms of cost, and do not necessarily have high processivity. Therefore, the methods using ion exchange resins are not suitable for industrial mass production.

As a method for producing a peptide mixture with a low phenylalanine content, a method using activated carbon which is based on hydrophobic interactions, and a method using a gel filter medium (gel filtration chromatography) which is a fractionation method based on a molecular weight are reported.

For example, Patent Literature 1 describes a method for producing a peptide mixture with a low phenylalanine content including hydrolyzing lactalbumin with one or two proteases (endopeptidase and exopeptidase) to obtain a peptide mixture, bringing the peptide mixture into contact with activated carbon to adsorb aromatic amino acid (phenylalanine) to the activated carbon, and removing the activated carbon along with the adsorbed aromatic amino acid (Examples 1 and 2).

Patent Literature 2 describes a method for producing a peptide mixture with a low phenylalanine content including 1) hydrolyzing a whey protein concentrate with a plurality of proteases (endopeptidase and exopeptidase) and collecting the hydrolyzed peptide mixture using a gel filter medium (gel filtration chromatography) (Examples 1 and 3), and 2) a series of procedural steps including hydrolyzing milk casein with a plurality of proteases and bringing the hydrolyzed peptide mixture into contact with activated carbon powder to adsorb phenylalanine to the activated carbon, and removing the activated carbon along with the adsorbed phenylalanine (Example 2).

In general, a soluble protein (also referred to as secretory protein) such as glycomacropeptide has a protein-folding structure containing hydrophobic amino acid residues (e.g., phenylalanine) in the interior of the molecule and hydrophilic amino acid residues on the molecular surface. This structure occurs frequently because when the soluble protein is soluble in an aqueous solution such as whey, the hydrophilic amino acid residue needs to be exposed on the molecular surface, which is an area of contact with the aqueous solution. Even when the soluble protein contains hydrophobic amino acid residues (e.g., phenylalanine), the hydrophobic amino acid residues have difficulty in becoming exposed on the molecular surface, which is an area of contact with the aqueous solution, and the hydrophobic amino acid residues are unlikely to interact with a hydrophobic carrier.

Therefore, a method based on hydrophobic interactions is not usually used in separation of the soluble protein. Instead, a method based on hydrophobic interactions is used in separation of highly hydrophobic low molecular compound such as steroid hormone, but not in separation of a soluble protein. For example, charcoal that is carbonaceous like activated carbon is used in preparation of a charcoal stripped serum. In the preparation of a charcoal stripped serum, a serum is brought into contact with charcoal, and the charcoal, to which a highly hydrophobic low molecular compound in the serum has been adsorbed, is then separated from the serum. This separation is performed in order to remove the highly hydrophobic low molecular compound from the serum while allowing a soluble protein such as a growth factor to remain in the serum as much as possible. Thus, there can be prepared a serum in which the soluble protein remains and the highly hydrophobic low molecular compound has been removed. That is, in the preparation of the charcoal stripped serum, the charcoal, which is carbonaceous, is mainly used for adsorption and separation of the highly hydrophobic low molecular compound, but not for adsorption and separation of the soluble protein. Currently, a method based on hydrophobic interactions is not usually used for separation of the soluble protein, as described above.

In the methods using activated carbon described in Patent Literatures 1 and 2, the hydrolysis step artificially induces a state where the peptide mixture containing a hydrophobic amino acid residue easily adsorbs to the activated carbon. That is, it is considered that when the soluble protein, which has difficulty in adsorbing to the activated carbon by hydrophobic interactions due to the hydrophobic amino acid residues (e.g., phenylalanine) being contained in the interior of the molecule, is hydrolyzed to form a small molecule, the soluble protein is converted into a peptide mixture that cannot have the aforementioned protein folding structure, and the hydrophobic amino acid residues become exposed on the molecular surface, which is an area of contact with the aqueous solution. However, according to the methods using activated carbon described in Patent Literatures 1 and 2, the hydrolysis step is necessary in the method for producing a peptide mixture with a low phenylalanine content. The method may be not only complicated, but also adversely affect properties such as flavor due to decomposition of a protein and the like by hydrolysis. The method using a gel filter medium described in Patent Literature 2 contains a problem in which substances having a similar molecular weight are unlikely to be separated.

### RELATED ART REFERENCE

### Patent Literature

Patent Literature 1: Japanese Examined Patent Publication No. H02-054070
Patent Literature 2: Japanese Patent No. 3682994

### Non-Patent Literature

Non-Patent Literature 1: J Food Sci. 2009; 74(4): E199-E206
Non-Patent Literature 2: Nakano and Ozimek, J Food Process Technol 2015, 7:1
Non-Patent Literature 3: Journal of Dairy Research (2018)85 110-113

### SUMMARY OF INVENTION

### PROBLEM TO BE SOLVED BY THE INVENTION

It is an object of the present invention to provide a method capable of easily producing high-purity glycomacropeptide with a low phenylalanine content on an industrial scale.

### MEANS FOR SOLVING PROBLEM

The inventors of the present invention have intensively studied, and as a result, found that when selecting a whey protein mixture containing glycomacropeptide and glycomacropeptide as a combination of a raw material and a substance to be purified, respectively, and using activated carbon, there can be easily produced high-purity glycomacropeptide with a very low phenylalanine content on an industrial scale.

In the method of the present invention, activated carbon that is easily available in a large amount and is inexpensive can be used to process a large amount of sample. Therefore, the method of the present invention is more suitable for industrial mass production than the methods of Non-Patent Literatures 1 to 3 using ion exchange resins. Unlike the methods of Non-patent Literatures 1 to 3 that use ion exchange resins and thus require an elution step with a salt solution, the method of the present invention does not require such an elution step. Therefore, the method of the present invention has an advantage in which a salt can be prevented from mixing in. Unlike the methods of Patent Literatures 1 and 2, the method of the present invention does not require a hydrolysis step. Therefore, the method is simple. Unlike the methods of Patent Literatures 1 and 2, the method of the present invention can produce glycomacropeptide (unhydrolyzed protein) instead of a hydrolyzed peptide, and an influence by hydrolysis on properties such as flavor can be avoided. The method of the present invention has an advantage in which the purity of glycomacropeptide can be increased even by just one simple process using activated carbon.

As far as the inventors are aware, a method for producing glycomacropeptide, a type of soluble protein by purification of glycomacropeptide with activated carbon has not yet been reported. Since such a method is contrary to common general knowledge about separation of soluble proteins described above, those skilled in the art cannot easily arrive at the method. Contrary to common general knowledge and unexpectedly, the inventors have found that activated carbon is highly compatible with a combination of a whey protein mixture containing glycomacropeptide (raw material) and glycomacropeptide (substance to be purified). Thus, the present invention has been completed.

That is, the present invention is as follows.
[1] A method for producing glycomacropeptide, comprising:
   (A) bringing a whey protein mixture containing glycomacropeptide into contact with activated carbon; and
   (B) separating the glycomacropeptide from the activated carbon.
[2] The method according to [1], wherein the whey protein mixture is crude glycomacropeptide derived from whey.
[3] The method according to [1] or [2], wherein a pore volume of the activated carbon is 0.50 mL/g or more.
[4] The method according to any one of [1] to [3], wherein the activated carbon is derived from the plant-based carbonaceous precursor.
[5] The method according to any one of [1] to [4], wherein an average pore diameter of the activated carbon is 2 nm or more.
[6] The method according to any one of [1] to [5], wherein a specific surface area of the activated carbon is 1,000 m²/g or more.
[7] The method according to any one of [1] to [6], wherein the whey protein mixture contains a protein containing a phenylalanine residue.
[8] The method according to any one of [1] to [7], wherein in step (B), separating glycomacropeptide with a phenylalanine concentration of 1,000 ppm by mass or less (in terms of solid content).

### EFFECT OF THE INVENTION

According to the method of the present invention, high-purity glycomacropeptide with a low phenylalanine content can be easily produced on an industrial scale.

### BRIEF DESCRIPTION OF DRAWINGS

FIG. 1 is a plot diagram of phenylalanine residual rate (vertical axis) and the pore volume of activated carbon (horizontal axis) during production of glycomacropeptide by a method according to an embodiment of the present invention.
FIG. 2 is a plot diagram of phenylalanine residual rate (vertical axis) and the average pore diameter of activated carbon (horizontal axis) during production of glycomacropeptide by the method according to the embodiment of the present invention.
FIG. 3 is a plot diagram of phenylalanine residual rate (vertical axis) and the amount of added activated carbon (horizontal axis) during production of glycomacropeptide by the method according to the embodiment of the present invention.
FIG. 4 is a plot diagram of phenylalanine residual rate (vertical axis) and the pH of a whey protein mixture (horizontal axis) during production of glycomacropeptide by the method according to the embodiment of the present invention.

### EMBODIMENT FOR CARRYING OUT THE INVENTION

A method for producing glycomacropeptide of the present invention includes the following:
(A) bringing a whey protein mixture containing glycomacropeptide into contact with activated carbon, and
(B) separating the glycomacropeptide from the activated carbon.

### -Step (A)-

In the step (A), a whey protein mixture containing glycomacropeptide is brought into contact with activated carbon. As a result, a phenylalanine component contained as an impurity in the whey protein mixture adsorbs to the activated carbon.

The whey protein mixture used in the step (A) is not particularly limited as long as it contains glycomacropeptide. Herein, "glycomacropeptide" (GMP) is a whey protein having a molecular weight of about 8,000, or in a case of containing glycosylation, a molecular weight of 25,000 to 30,000, and this protein contains no phenylalanine residues in the amino acid sequence when it is a pure substance, as described above.

Examples of the whey protein mixture used in the step (A) may include whey obtained during production of processed milk products such as cheese, a concentrate thereof, and water-dilution thereof. Alternatively, crude glycomacropeptide derived from whey may be used as the whey protein mixture. In the present invention, the "crude glycomacropeptide derived from whey" refers to crudely purified glycomacropeptide containing a phenylalanine component as an impurity that is obtained from whey by industrial separation and purification. Such crudely purified glycomacropeptide may be commercially referred to as "purified glycomacropeptide." A method for obtaining crude glycomacropeptide by industrially separating and purifying glycomacropeptide from whey has been publicly known. For example, a method for obtaining crude glycomacropeptide from whey by ion exchange chromatography or ultrafiltration has been known (U.S. Patent No. 5,968,586, etc.). The isoelectric point of glycomacropeptide is about 3.8, which is lower than the isoelectric point of other major whey proteins (usually higher than 4.3). Such a difference in the isoelectric point is useful when obtaining crude glycomacropeptide by industrially separating and purifying glycomacropeptide from whey. However, there is a limit to separation of glycomacropeptide from a protein containing phenylalanine residues. In the present invention, when crude glycomacropeptide derived from whey is used as the whey protein mixture, crude glycomacropeptide obtained at any stage in a process of industrially separating and purifying glycomacropeptide from whey may be used. When the solid content in the crude glycomacropeptide is low, a concentrate thereof may be used. When the solid content in the crude glycomacropeptide is high, water-dilution threreof may be used.

The solid content concentration of the whey protein mixture used in the step (A) depends on a specific aspect of a treatment for contacting with activated carbon (batch or continuous treatment, etc.), and is preferably 10% by mass or less, 8% by mass or less, 6% by mass or less, or 5% by mass or less. The lower limit of the solid content concentration is not particularly limited, and is preferably 0.1% by mass or more, 0.2% by mass or more, 0.3% by mass or more, or 0.5% by mass or more, from the viewpoint of process efficiency.

The whey protein mixture used in the step (A) contains a phenylalanine component (e.g., free phenylalanine, and a peptide and a protein containing phenylalanine residues) as an impurity. The phenylalanine concentration (in terms of solid content) in the whey protein mixture is not particularly limited, and is preferably 10,000 ppm by mass or less, 8,000 ppm by mass or less, 6,000 ppm by mass or less, 5,000 ppm by mass or less, 4,000 ppm by mass or less, or 3,000 ppm by mass or less, from the viewpoint of obtaining glycomacropeptide with a low phenylalanine content. The lower limit of the phenylalanine concentration (in terms of solid content) may be usually more than 1,000 ppm by mass, 1,100 ppm by mass or more, 1,200 ppm by mass or more, 1,300 ppm by mass or more, or 1,400 ppm by mass or more. Herein, the phenylalanine concentration (in terms of solid content) in the whey protein mixture refers to the proportion (ppm by mass) of the total amount of free phenylalanine and phenylalanine residues (free phenylalanine equivalent) relative to the whole solid content in the whey protein mixture. The phenylalanine concentration may be measured, for example, by (1) a method in which the whey protein mixture is subjected to an acid treatment at high temperatures (e.g., at 110°C for 22 hours in the presence of 6 N hydrochloric acid) to hydrolyze a peptide component into free amino acid, and the free phenylalanine in the free amino acid is quantified (e.g., high performance liquid chromatography, mass spectrometer, or amino acid analyzer), or (2) a method in which the absorbance is measured at an absorption wavelength (260 nm) specific to the phenylalanine residue in the whey protein mixture, and the phenylalanine content is measured using the absorbance as an indication. In the measurements, a phenylalanine authentic sample may be used. In particular, the phenylalanine concentration in the whey protein mixture can be measured in accordance with a method described below in [Measurement of Phenylalanine Concentration].

The whey protein mixture used in the step (A) may contain a component usually contained in whey in addition to glycomacropeptide and the phenylalanine component. Further, the whey protein mixture may contain additives such as a pH adjuster or a defoamer so long as not impairing the advantageous effects of the present invention.

In an embodiment, the whey protein mixture used in the step (A) is whey or water-dilution thereof. In another embodiment, the whey protein mixture used in the step (A) is crude glycomacropeptide derived from whey. The properties of the whey protein mixture such as phenylalanine concentration are as described above.

In an embodiment, the whey protein mixture contains a protein containing a phenylalanine residue (hereinafter also referred to as "Phe-containing protein") as an impurity phenylalanine component. In the present invention, a phenylalanine contaminant including the Phe-containing protein can be selectively and easily removed from the whey protein mixture, which significantly contributes to easy production of high-purity glycomacropeptide with a low phenylalanine content on an industrial scale.

The activated carbon used in the step (A) will be described below.

The shape of the activated carbon is not particularly limited, and for example, the activated carbon may be granular, fibrous (thread shape, cloth shape, and felt shape), or the like shape. Specifically, the shape of the activated carbon can be appropriately selected according to use application. In particular, granular activated carbon is preferable since the adsorption capacity of the phenylalanine component per unit volume is high. In the case of a granular carbonaceous material, the size thereof is not particularly limited, and specifically, the particle size thereof may be appropriately adjusted according to use application.

A raw material for the activated carbon (carbonaceous precursor) is not particularly limited, and examples thereof may include a plant-based carbonaceous precursor (for example, materials derived from plants including wood, wood waste such as sawdust and wood shavings, wood charcoal, fruit shells such as a coconut shell and a walnut shell, fruit seeds, by-products of pulp production, lignin, and molasses), a mineral-based carbonaceous precursor (for example, materials derived from minerals such as peat, lignite, brown coal, bituminous coal, anthracite coal, coke, coal tar, coal tar pitch, petroleum distillation residue, and petroleum pitch), a synthetic resin-based carbonaceous precursor (for example, materials derived from synthetic resins such as a phenolic resin, polyvinylidene chloride, and an acrylic resin), and a natural fiber-based carbonaceous precursor (for example, materials derived from natural fibers including natural fibers such as cellulose and regenerated fibers such as rayon). Among these, the plant-based carbonaceous precursor is preferable since such a material easily forms activated carbon capable of selectively adsorbing the phenylalanine component (in particular, the Phe-containing protein) in the whey protein mixture. Therefore, in a suitable embodiment, the activated carbon is derived from the plant-based carbonaceous precursor. From the viewpoint of achieving activated carbon capable of further efficiently adsorbing and removing the phenylalanine component in the whey protein mixture, the raw material is preferably wood, wood waste, or fruit shells, and more preferably wood or wood waste. Therefore, in a particularly suitable embodiment, wood or wood waste is used as the plant-based carbonaceous precursor.

From the viewpoint of selectively adsorbing the phenylalanine component in the whey protein mixture, the pore volume of the activated carbon is preferably 0.40 mL/g or more, 0.45 mL/g or more, 0.50 mL/g or more, 0.55 mL/g or more, 0.60 mL/g or more, 0.65 mL/g or more, or 0.70 mL/g or more. The inventors have found that the pore volume of the activated carbon closely correlates with capacity for selectively adsorbing the phenylalanine component. The inventors have confirmed that the activated carbon that has a pore volume of 0.6 mL/g or more (in particular, activated carbon derived from the plant-based carbonaceous precursor) is particularly excellent in terms of capacity for selectively adsorbing the phenylalanine component in the whey protein mixture. The upper limit of the pore volume of the activated carbon is not particularly limited, and from the viewpoint of mechanical strength, may be usually 1.7 mL/g or less, 1.5 mL/g or less, or 1.3 mL/g or less. The pore volume (total pore volume) of the activated carbon can be determined by the Gurvish rule from the nitrogen adsorption amount when the relative pressure P/P0 on a nitrogen adsorption isotherm is 0.99.

From the viewpoint of selectively adsorbing the phenylalanine component in the whey protein mixture, the average pore diameter of the activated carbon is preferably 1.9 nm or more, 2 nm or more, 2.1 nm or more, 2.2 nm or more, 2.3 nm or more, 2.4 nm or more, or 2.5 nm or more. The inventors have found that the average pore diameter of the activated carbon closely correlates with the capacity of selectively adsorbing the phenylalanine component. The inventors have confirmed that the activated carbon that has an average pore diameter of 2 nm or more (in particular, activated carbon derived from the plant-based carbonaceous precursor) is particularly excellent in terms of the capacity of selectively adsorbing the phenylalanine component in the whey protein mixture. The upper limit of the average pore diameter of the activated carbon is not particularly limited, and from the viewpoint of mechanical strength, may be usually 5 nm or less, 4.5 nm or less, 4 nm or less, 3.5 nm or less, or 3 nm or less. The average pore diameter of the activated carbon can be calculated from the nitrogen adsorption isotherm. Specifically, the average pore diameter of the activated carbon may be determined by an expression: 4000 × [pore volume (mL/g) described above/specific surface area (m²/g) described below].

From the viewpoint of selectively adsorbing the phenylalanine component in the whey protein mixture, the specific surface area of the activated carbon is preferably 900 m²/g or more, 950 m²/g or more, 1,000 m²/g or more, 1,050 m²/g or more, or 1,100 m²/g or more. The upper limit of the specific surface area of the activated carbon is not particularly limited, and may be usually 2,000 m²/g or less, 1,800 m²/g or less, or 1,600 m²/g or less. The specific surface area of the activated carbon may be calculated from the nitrogen adsorption isotherm by a BET theory.

In a suitable embodiment, the activated carbon used in the step (A) satisfies the following condition (i) and at least one of the following conditions (ii) and (iii) :
(i) the activated carbon is derived from the plant-based carbonaceous precursor;
(ii) the pore volume is 0.5 mL/g or more; and
(iii) the average pore diameter is 2 nm or more.

In a more suitable embodiment, the activated carbon used in the step (A) satisfies all the conditions (i), (ii), and (iii). In the suitable embodiments, a suitable type of carbonaceous precursor and suitable ranges of the pore volume, the average pore diameter, and the like are as described above.

The activated carbon used in the step (A) can be produced by activation of the aforementioned carbonaceous precursor. When carbonization is required prior to the activation, oxygen or air may be usually blocked followed by carbonization, for example, at 400 to 800°C (preferably 500 to 800°C, and more preferably 550 to 750°C). In this case, a carbonaceous material may be produced by activation of a raw material charcoal, which is obtained by carbonization of the carbonaceous precursor.

A method for activation is not particularly limited as long as it can activate the carbonaceous precursor. The activation method may be chemical activation with an activator such as zinc chloride or phosphoric acid or gas activation with an activation gas such as a gas containing water vapor or carbon dioxide or air, or a combination of chemical activation and gas activation. The activation method may be appropriately selected according to the type of the carbonaceous precursor, and the like. Conditions for activation are publicly known in the field of activated carbon manufacturing. In a suitable aspect, the plant-based carbonaceous precursor is subjected to gas activation, to obtain activated carbon. Since the activation gas contains water vapor or carbon dioxide in an appropriate amount, a combustion gas of hydrocarbon (e.g., a light gas such as methane, propane, or butane, and a liquid fuel such as light oil, kerosene, or heavy oil) is preferably used as the activation gas. The water vapor concentration in the activation gas is preferably 10 to 40% by volume. The temperature during gas activation may be 700 to 1,000°C (preferably 800°C to 1, 000°C or 850°C to 950°C) . The activation time may be any amount of time enough to secure desired properties such as the pore volume, the average pore diameter, and the specific surface area. When the degree of activation advancement is adjusted according to the type of carbonaceous precursor (the suitable type is as described above), activated carbon having a pore volume, an average pore diameter, and a specific surface area falling within the suitable ranges can be obtained.

In the step (A), as the activated carbon, one type thereof may be solely used, and two or more types thereof may be used in combination. When two or more types of activated carbon are used, it is preferable that a mixture thereof as a whole has the desired properties such as the pore volume, the average pore diameter, and the specific surface area.

In the step (A), a treatment for contact of the whey protein mixture with the activated carbon is not particularly limited as long as the whey protein mixture can be brought into contact with the activated carbon. The treatment may be a batch contact treatment or a continuous contact treatment. For example, in the batch contact treatment, the whey protein mixture and the activated carbon may be fed in an adsorption tower, and be brought into contact with each other. The treatment at that time may be a fluidized bed treatment in which the activated carbon is brought into contact with the whey protein mixture with the activated carbon suspended in the whey protein mixture, or a fixed bed treatment in which the activated carbon is brought into contact with the whey protein mixture with the activated carbon fixed on a carrier. In the continuous contact treatment, the whey protein mixture may be passed through a filter filled with the activated carbon, to continuously come into contact with the activated carbon. Various techniques for bringing an adsorbent into contact with a liquid to be treated are known. Among the known techniques, any contact technique may be used to perform the step (A) of the present invention.

From the viewpoint of achieving smooth contact of the whey protein mixture with the activated carbon, the temperature of the whey protein mixture during contact with the activated carbon in the step (A) is preferably 10°C or higher, 15°C or higher, or 20°C or higher. From the viewpoint of producing high-purity glycomacropeptide containing a small amount of phenylalanine component, a higher temperature is suitable, and the temperature is preferably 25°C or higher, and may be as high as 30°C or higher, 35°C or higher, 40°C or higher, or 45°C or higher. The upper limit of the temperature is not particularly limited, and may be 85°C or lower, or 80°C or lower in practical terms. In a suitable embodiment, the temperature of the whey protein mixture during contact with the activated carbon in the step (A) is 20°C to 80°C (more preferably 25°C to 70°C, 25°C to 60°C, or 25°C to 50°C) from the viewpoint of producing high-purity glycomacropeptide with a low phenylalanine content with a high yield.

The pH (25°C) of the whey protein mixture during contact with the activated carbon in the step (A) may fall within a range of 3 to 9 (preferably a range of 4 to 8). According to the method of the present invention, the phenylalanine component in the whey protein mixture may be selectively removed without particularly adjusting the pH of the whey protein mixture.

The time for contact of the whey protein mixture with the activated carbon in the step (A) may be appropriately determined according to various contact conditions such as the phenylalanine concentration in the whey protein mixture, properties of the activated carbon, temperature, and linear speed (in the continuous treatment). It is preferable that the breakthrough time is measured in advance under an adopted contact condition and the upper limit of the contact time is set to be such the breakthrough time. Herein, the "breakthrough time" refers to a time that it takes for the activated carbon to reach adsorption saturation.

The amount of the activated carbon in the step (A) is not particularly limited as long as the object of the present invention is achieved. From the viewpoint of obtaining glycomacropeptide with a low phenylalanine content, the amount of the activated carbon in the step (A) on a dry weight (dry mass) basis relative to a whole solid content of 100% by mass in the whey protein mixture is preferably 10% by mass or more, 15% by mass or more, 20% by mass or more, 25% by mass or more, or 30% by mass or more. The upper limit of the amount of the activated carbon may be, for example, 500% by mass or less, 400% by mass or less, 300% by mass or less, or the like, and preferably 200% by mass or less, 100% by mass or less, 80% by mass or less, or 60% by mass or less. When the step (A) is performed by the continuous contact treatment, a liquid flow condition is not particularly limited. From the viewpoint of obtaining high-purity glycomacropeptide with a low phenylalanine content so that the pressure loss does not become extremely large, the step (A) may be performed at a spatial velocity (SV) of, preferably 200 to 10,000/hr, and more preferably 500 to 8,000/hr.

### -Step (B)-

In the step (B), glycomacropeptide is separated from the activated carbon. Since the phenylalanine component that has adsorbed to the activated carbon is separated and removed along with the activated carbon, glycomacropeptide with a low phenylalanine content is obtained.

When the fluidized bed treatment is adopted in the step (A), the separation of glycomacropeptide from the activated carbon may be performed by (1) leaving a suspension standing after the contact treatment to precipitate the activated carbon and subsequently collecting a supernatant (containing glycomacropeptide), or (2) separating the suspension after the contact treatment into the activated carbon and a liquid phase (containing glycomacropeptide) by a separation treatment such as filtration or centrifugal separation, or by a combination of the treatments (1) and (2). When the fixed bed treatment is adopted in the step (A), a liquid phase (containing glycomacropeptide) after the contact treatment can be collected. In order to increase the yield of glycomacropeptide with a low phenylalanine content, the method may further include a treatment in which the glycomacropeptide attached to the activated carbon is washed with water (to obtain glycomacropeptide with a low phenylalanine content). Hereinafter, the liquid phase containing glycomacropeptide separated from the activated carbon in the step (B) is also referred to as "glycomacropeptide separation phase."

When a phenylalanine concentration in the whey protein mixture used in the step (A) is represented by C₀ and a phenylalanine concentration in the glycomacropeptide separation phase obtained in the step (B) is represented by C₁, C₀ and C₁ satisfy preferably a relationship of C₁/C₀ ≤ 0.7, and more preferably a relationship of C₁/C₀ ≤0.6 or C₁/C₀ ≤0.5. When a step (C) described below is performed, the previous sentence can be applied by replacing "phenylalanine concentration in the glycomacropeptide separation phase obtained in the step (B)" with "phenylalanine concentration in glycomacropeptide isolated in the step (C)."

The phenylalanine concentration (in terms of solid content) in the glycomacropeptide separation phase obtained in the step (B) is preferably 1,200 ppm by mass or less, 1,100 ppm by mass or less, or 1,000 ppm by mass or less. When the suitable activated carbon described above is used, the phenylalanine concentration (in terms of solid content) can be further decreased, and for example, a phenylalanine concentration as low as 900 ppm by mass or less, 800 ppm by mass or less, 700 ppm by mass or less, 600 ppm by mass or less, 500 ppm by mass or less, 400 ppm by mass or less, 300 ppm by mass or less, or 200 ppm by mass or less can be achieved. The lower limit of the phenylalanine concentration (in terms of solid content) is preferably as low as possible without particular limitation, and may be usually 10 ppm by mass or more, 50 ppm by mass or more, 100 ppm by mass or more, or the like. When the step (C) described below is performed, the previous sentences can be applied by replacing "phenylalanine concentration (in terms of solid content) in the glycomacropeptide separation phase obtained in the step (B)" with "phenylalanine concentration (in terms of solid content) in glycomacropeptide isolated in the step (C)."

### -Other Steps-

The method for producing glycomacropeptide of the present invention may further include another step that is useful in production of high-purity glycomacropeptide with a low phenylalanine content from the whey protein mixture as long as the method includes the steps (A) and (B). In the step (A), for example, the whey, the concentrate thereof, or the water-dilution thereof; or the crude glycomacropeptide obtained at any stage in a process of industrially separating and purifying glycomacropeptide from the whey is used as the whey protein mixture. In this process, a contaminant such as other whey protein and whey peptide, and free amino acid (hereinafter sometimes simply referred to as "other contaminant") may be present as well as glycomacropeptide and the phenylalanine component in the whey protein mixture. Therefore, when the whey protein mixture used in the step (A) contains the other contaminant, a suitable and publicly known treatment may be performed for separation of glycomacropeptide from the other contaminant. Examples of such a treatment may include publicly known treatments that are conventionally performed for industrial separation and purification of glycomacropeptide from the whey, for example, separation treatments such as ion exchange chromatography and ultrafiltration. With the treatment, procedural steps associated with the treatment (e.g., procedural steps for hydrolysis of the whey protein using an enzyme, adjustment of PH, and the like) may be performed as long as they do not impair the advantageous effects of the present invention. Accordingly, in an embodiment, the method for producing glycomacropeptide of the present invention may further include a step (C) isolating glycomacropeptide from the glycomacropeptide separation phase obtained in the step (B) .

The method for producing glycomacropeptide of the present invention may further include adjusting the solid content concentration in an obtained final glycomacropeptide product. For example, a sample having a desired glycomacropeptide concentration may be prepared by diluting the glycomacropeptide product with water or concentrating the glycomacropeptide product, or a solid sample (for example, dried or semi-dried powder) may be prepared by removing water content from the glycomacropeptide product.

### Example

Hereinafter, the present invention will be described in more detail by illustrating examples. However, the present invention is not limited to the following examples. In the following description, "%" and "ppm" mean "% by mass" and "ppm by mass", respectively, unless otherwise specified.

### <Evaluation method>

Each physical property value in the example was measured by the method shown below.

### [Measurement of phenylalanine concentration]

A pressure-resistant glass tube was charged with about 100 mg (dry weight basis) of a whey protein mixture, and further about 20 mL of 6-N hydrochloric acid, and then was sealed and heated at 110°C for 22 hours. After completion of heating, hydrochloric acid was distilled off under a reduced pressure. The residue was diluted with 0.02-N hydrochloric acid into 100 mL, and the quantity of phenylalanine was determined by high performance liquid chromatography. Specifically, the quantity of phenylalanine was determined using an amino acid mixture standard solution H type available from Fujifilm Wako Pure Chemical Corporation as a standard solution with L-8900 Amino Acid Analyzer (physiological fluid analysis mode) manufactured by Hitachi, Ltd.

For the whey protein mixtures before and after contact with activated carbon, the measurement was performed.

### [Measurement of pH]

The pH (25°C) of a whey protein mixture was measured using a phthalic acid pH standard solution with a pH of 4.01 (25°C) and a neutral phosphate pH standard solution with a pH of 6.86 (25°C) available from DKK-TOA Corporation with LAQUA pH/ION METER F-73 manufactured by HORIBA, Ltd.

### <Activated carbon>

Compositions of activated carbons A to I used in Examples are shown in Table 1. The activated carbons A to I were produced by activation through activation methods described Table 1 using carbonaceous precursors described in Table 1 until the pore volume, the average pore diameter, and the specific surface area reached respective values in Table 1.

### [Table 1]

**(TABLE 1)**

| | ACTIVATED CARBON | | | | | | | | |
|---|---|---|---|---|---|---|---|---|---|
| | A | B | C | D | E | F | G | H | I |
| CARBONACEOUS PRECURSOR | PLANT (EUCALYPTUS TREE ROOT) | PLANT (BAMBOO) | MINERAL (COAL) | MINERAL (COAL) | PLANT (SAWDUST) | PLANT (COCONUT SHELL) | PLANT (WOOD) | PLANT (WOOD) | PLANT (WOOD) |
| ACTIVATION METHOD | GAS (WATER VAPOR) | GAS (WATER VAPOR) | GAS (WATER VAPOR) | GAS (WATER VAPOR) | CHEMICAL (PHOSPHORIC ACID) | GAS (WATER VAPOR) | CHEMICAL (PHOSPHORIC ACID) | GAS (WATER VAPOR) | CHEMICAL +GAS (PHOSPHORIC ACID +WATER VAPOR) |
| PORE VOLUME [mL/g] | 0.789 | 0.496 | 0.438 | 0.633 | 1.179 | 0.516 | 1.52 | 0.80 | 1.27 |
| AVERAGE PORE DIAMETER [nm] | 2.48 | 1.75 | 2.21 | 2.54 | 3.11 | 1.87 | 3.98 | 3.29 | 3.60 |
| SPECIFIC SURFACE AREA [m²/g] | 1289 | 1136 | 792 | 983 | 1515 | 1104 | 1523 | 972 | 1414 |

### [Example 1]

### 1-1. Production of glycomacropeptide

### <Production Example 1>

As a whey protein mixture, an aqueous composition was prepared in which 1.1 g of purified glycomacropeptide (GMP) powder (available from Agropur, GMP 97.1% of protein, Moisture 6.9%, phenylalanine 1454 ppm/whole solid content) was dissolved in 49 mL of ultrapure water. To the whey protein mixture, 400 mg (dry weight basis) of the activated carbon A was added, and the mixture was stirred at room temperature of 25°C for 1 hour. Subsequently, the activated carbon suspension was filtered through a filter, to obtain about 48 g of a clear GMP treated liquid. The obtained treated liquid was dried under heating at 105°C, to obtain about 0.91 g (phenylalanine 883 ppm/whole solid content) of GMP powder with a low phenylalanine content.

### <Production Example 2>

A whey protein mixture was prepared by the same manner as that of Production Example 1. To the whey protein mixture, 400 mg (dry weight basis) of the activated carbon B was added, and the mixture was stirred at room temperature of 25°C for 1 hour. Subsequently, the activated carbon suspension was filtered through a filter, to obtain about 47 g of a clear GMP treated liquid. The obtained treated liquid was dried under heating at 105°C, to obtain about 0.93 g (phenylalanine 1222 ppm/whole solid content) of GMP powder with a low phenylalanine content.

### <Production Example 3>

A whey protein mixture was prepared by the same manner as that of Production Example 1. To the whey protein mixture, 400 mg (dry weight basis) of the activated carbon C was added, and the mixture was stirred at room temperature of 25°C for 1 hour. Subsequently, the activated carbon suspension was filtered through a filter, to obtain about 48 g of a clear GMP treated liquid. The obtained treated liquid was dried under heating at 105°C, to obtain about 0.95 g (phenylalanine 1217 ppm/whole solid content) of GMP powder with a low phenylalanine content.

### <Production Example 4>

A whey protein mixture was prepared by the same manner as that of Production Example 1. To the whey protein mixture, 400 mg (dry weight basis) of the activated carbon D was added, and the mixture was stirred at room temperature of 25°C for 1 hour. Subsequently, the activated carbon suspension was filtered through a filter, to obtain about 48 g of a clear GMP treated liquid. The obtained treated liquid was dried under heating at 105°C, to obtain about 0.93 g (phenylalanine 1049 ppm/whole solid content) of GMP powder with a low phenylalanine content.

### <Production Example 5>

A whey protein mixture was prepared by the same manner as that of Production Example 1. To the whey protein mixture, 400 mg (dry weight basis) of the activated carbon E was added, and the mixture was stirred at room temperature of 25°C for 1 hour. Subsequently, the activated carbon suspension was filtered through a filter, to obtain about 47 g of a clear GMP treated liquid. The obtained treated liquid was dried under heating at 105°C, to obtain about 0.84 g (phenylalanine 619 ppm/whole solid content) of GMP powder with a low phenylalanine content.

### <Production Example 6>

A whey protein mixture was prepared by the same manner as that of Production Example 1. To the whey protein mixture, 400 mg (dry weight basis) of the activated carbon F was added, and the mixture was stirred at room temperature of 25°C for 1 hour. Subsequently, the activated carbon suspension was filtered through a filter, to obtain about 48 g of a clear GMP treated liquid. The obtained treated liquid was dried under heating at 105°C, to obtain about 0.95 g (phenylalanine 1189 ppm/whole solid content) of GMP powder with a low phenylalanine content.

### <Production Example 7 (Control)>

A whey protein mixture was prepared by the same manner as that of Production Example 1. By the same manner as that of Production Example 1 except that the activated carbon was not added, about 1.0 g (phenylalanine 1,454 ppm/whole solid content) of GMP powder was then obtained.

### <Production Example 8>

As a whey protein mixture, an aqueous composition was prepared in which 1.3 g of purified glycomacropeptide (GMP) powder (available from Agropur, GMP 97.1% of protein, Moisture 6.9%, phenylalanine 1454 ppm/whole solid content) was dissolved in 59 mL of ultrapure water. To the whey protein mixture, 480 mg (dry weight basis) of the activated carbon G was added, and the mixture was stirred at room temperature of 25°C for 1 hour. Subsequently, the activated carbon suspension was filtered through a filter, to obtain about 58 g of clear GMP treated liquid. The obtained treated liquid was dried under heating at 105°C, to obtain about 1.08 g (phenylalanine 581 ppm/whole solid content) of GMP powder with a low phenylalanine content.

### <Production Example 9>

A whey protein mixture was prepared by the same manner as that of Production Example 8. To the whey protein mixture, 480 mg (dry weight basis) of the activated carbon H was added, and the mixture was stirred at room temperature of 25°C for 1 hour. Subsequently, the activated carbon suspension was filtered through a filter, to obtain about 58 g of a clear GMP treated liquid. The obtained treated liquid was dried under heating at 105°C, to obtain about 1.10 g (phenylalanine 1077 ppm/whole solid content) of GMP powder with a low phenylalanine content.

### <Production Example 10>

A whey protein mixture was prepared by the same manner as that of Production Example 8. To the whey protein mixture, 480 mg (dry weight basis) of the activated carbon I was added, and the mixture was stirred at room temperature of 25°C for 1 hour. Subsequently, the activated carbon suspension was filtered through a filter, to obtain about 58 g of a clear GMP treated liquid. The obtained treated liquid was dried under heating at 105°C, to obtain about 1.10 g (phenylalanine 779 ppm/whole solid content) of GMP powder with a low phenylalanine content.

### <Production Example 11>

A whey protein mixture was prepared by the same manner as that of Production Example 8. To the whey protein mixture, 240 mg (dry weight basis) of the activated carbon I was added, and the mixture was stirred at room temperature of 25°C for 1 hour. Subsequently, the activated carbon suspension was filtered through a filter, to obtain about 58 g of a clear GMP treated liquid. The obtained treated liquid was dried under heating at 105°C, to obtain about 1.15 g (phenylalanine 1071 ppm/whole solid content) of GMP powder with a low phenylalanine content.

### <Production Example 12>

A whey protein mixture was prepared by the same manner as that of Production Example 8. To the whey protein mixture, 720 mg (dry weight basis) of the activated carbon I was added, and the mixture was stirred at room temperature of 25°C for 1 hour. Subsequently, the activated carbon suspension was filtered through a filter, to obtain about 58 g of a clear GMP treated liquid. The obtained treated liquid was dried under heating at 105°C, to obtain about 1.08 g (phenylalanine 594 ppm/whole solid content) of GMP powder with a low phenylalanine content.

### <Production Example 13>

As a whey protein mixture, an aqueous composition was prepared in which 1.8 g of purified glycomacropeptide (GMP) powder (available from Agropur, GMP 97.1% of protein, Moisture 6.9%, phenylalanine 1454 ppm/whole solid content) was dissolved in 78 mL of ultrapure water. To the whey protein mixture, 1,440 mg (dry weight basis) of the activated carbon I was added, and the mixture was stirred at room temperature of 25°C for 1 hour. Subsequently, the activated carbon suspension was filtered through a filter, to obtain about 77 g of a clear GMP treated liquid. The obtained treated liquid was dried under heating at 105°C, to obtain about 1.29 g (phenylalanine 396 ppm/whole solid content) of GMP powder with a low phenylalanine content.

### <Production Example 14>

A whey protein mixture was prepared by the same manner as that of Production Example 13. To the whey protein mixture, 2880 mg (dry weight basis) of the activated carbon I was added, and the mixture was stirred at room temperature of 25°C for 1 hour. Subsequently, the activated carbon suspension was filtered through a filter, to obtain about 74 g of a clear GMP treated liquid. The obtained treated liquid was dried under heating at 105°C, to obtain about 1.05 g (phenylalanine 216 ppm/whole solid content) of GMP powder with a low phenylalanine content.

### <Production Example 15>

A whey protein mixture was prepared by the same manner as that of Production Example 13. To the whey protein mixture, 5440 mg (dry weight basis) of the activated carbon I was added, and the mixture was stirred at room temperature of 25°C for 1 hour. Subsequently, the activated carbon suspension was filtered through a filter, to obtain about 69 g of a clear GMP treated liquid. The obtained treated liquid was dried under heating at 105°C, to obtain about 0.67 g (phenylalanine 116 ppm/whole solid content) of GMP powder with a low phenylalanine content.

### 1-2. Correlation between properties of activated carbon and phenylalanine (Phe) residual rate

Properties of the activated carbons and the Phe residual rates in Production Examples 1 to 10 are shown in Table 2. The lower Phe residual rate indicates that the Phe component in the whey protein mixture is selectively removed. The Phe residual rate (%) was calculated by Expression: [(C₁/C₀)/(C₂/C₀)] × 100, that is, Expression: [C₁/C_{2]} × 100, wherein C₀ [ppm] was the Phe concentration in the whey protein mixture before the treatment with the activated carbon, and C₁ [ppm] was the Phe concentration in the whey protein mixture after the treatment with the activated carbon, and C₂ [ppm] was the Phe concentration in the whey protein mixture that is the control.

### [Table 2]

**(TABLE 2)**

| | | PRODUCTION EXAMPLE | | | | | | | | |
|---|---|---|---|---|---|---|---|---|---|---|
| | | 1 | 2 | 3 | 4 | 5 | 6 | 8 | 9 | 10 |
| ACTIVATED CARBON | No. | A | B | C | D | E | F | G | H | I |
| | PORE VOLUME [mL/g] | 0.789 | 0.496 | 0.438 | 0.633 | 1.179 | 0.516 | 1.52 | 0.80 | 1.27 |
| | AVERAGE PORE DIAMETER [nm] | 2.48 | 1.75 | 221 | 2.54 | 3.11 | 1.87 | 3.98 | 3.29 | 3.60 |
| | SPECIFIC SURFACE AREA [m²/g] | 1289 | 1136 | 792 | 983 | 1515 | 1104 | 1523 | 972 | 1414 |
| C₁ [ppm]^{∗1} | | 883 | 1222 | 1217 | 1049 | 619 | 1189 | 581 | 1077 | 779 |
| C₂ [ppm]^{∗2} | | 1454 | 1454 | 1454 | 1454 | 1454 | 1454 | 1454 | 1454 | 1454 |
| Phe RESIDUAL RATE [%] | | 60.7 | 84.0 | 83.7 | 72.1 | 42.6 | 81.8 | 40.0 | 74.1 | 53.6 |

| | | | | | | | | | | |
|---|---|---|---|---|---|---|---|---|---|---|
| ^{∗1} Phe CONCENTRATION IN SAMPLE AFTER TREATMENT WITH ACTIVATED CARBON ^{∗2} Phe CONCENTRATION IN CONTROL SAMPLE | | | | | | | | | | |

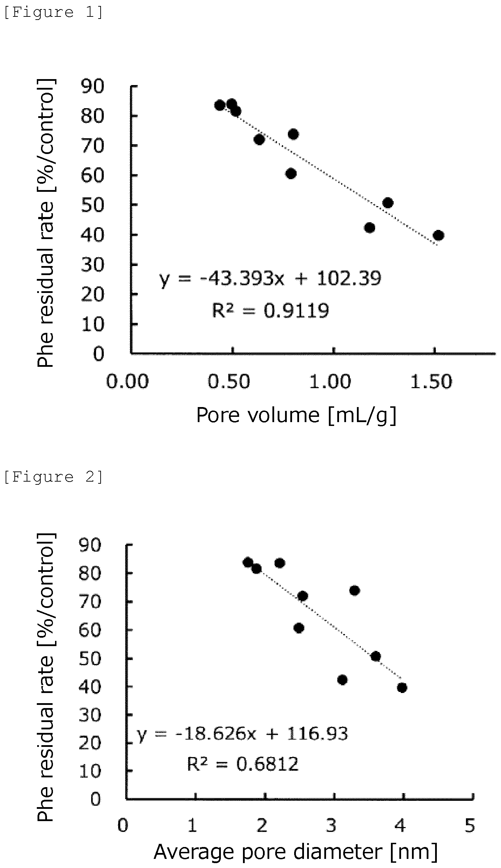

It was confirmed that the Phe residual rate tended to decrease as the value of pore volume increased. It was also confirmed that the Phe residual rate tended to decrease as the average pore diameter increased. FIG. 1 is a graph in which the pore volume (horizontal axis) and the Phe residual rate (vertical axis) are plotted. FIG. 2 is a graph in which the average pore diameter (horizontal axis) and the Phe residual rate (vertical axis) are plotted. FIGs. 1 and 2 illustrate an approximate line based on a least-square method, an expression thereof, and a coefficient of determination R² together. In particular, the coefficient of determination R² for the pore volume was more than 0.91, and it was confirmed that the correlation between the pore volume and the Phe residual rate was very close.

1-3. Correlation between amount of activated carbon and Phe residual rate

The amounts of the added activated carbon and the Phe residual rates in Production Examples 10 to 15 using the activated carbon I are shown in Table 3. The Phe residual rates were calculated by the same manner as that of 1-2 described above.

### [Table 3]

**(TABLE 3)**

| | | PRODUCTION EXAMPLE | | | | | |
|---|---|---|---|---|---|---|---|
| | | 11 | (10) | 12 | 13 | 14 | 15 |
| ACTIVATED CARBON | No. | I | I | I | I | I | I |
| | PORE VOLUME [mL/g] | 1.27 | 1.27 | 1.27 | 1.27 | 1.27 | 1.27 |
| | AVERAGE PORE DIAMETER [nm] | 3.60 | 3.60 | 3.60 | 3.60 | 3.60 | 3.60 |
| | SPECIFC SURFACE AREA [m²/g] | 1414 | 1414 | 1414 | 1414 | 1414 | 1414 |
| AMOUNT OF ADDED ACTIVATED CARBON [%]^{∗} | | 20 | 40 | 60 | 90 | 180 | 360 |
| C₁ [ppm]^{∗1} | | 1071 | 779 | 594 | 396 | 216 | 116 |
| C₂ [ppm]^{∗2} | | 1454 | 1454 | 1454 | 1454 | 1454 | 1454 |
| Phe RESIDUAL RATE [%] | | 73.7 | 53.6 | 40.9 | 27.2 | 14.9 | 8.0 |

| | | | | | | | |
|---|---|---|---|---|---|---|---|
| ^{∗} VALUE (% BY MASS) RELATIVE TO WHOLE SOLID CONTENT OF 100% BY MASS IN WHEY PROTEIN MIXTURE ^{*1} Phe CONCENTRATION IN SAMPLE AFTER TREATMENT WITH ACTIVATED CARBON ^{∗2} Phe CONCENTRATION IN CONTROL SAMPLE | | | | | | | |

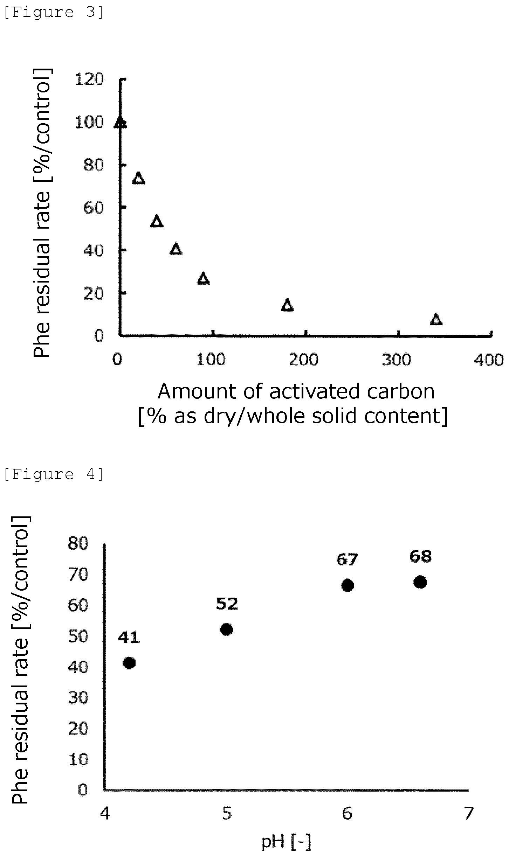

It was confirmed that the Phe residual rate in the sample tended to decrease as the amount of added activated carbon increased. FIG. 3 is a graph in which the amount of added activated carbon (horizontal axis) and the Phe residual rate (vertical axis) are plotted.

### [Example 2]

### 2-1. Production of glycomacropeptide

As a whey protein mixture, an aqueous composition was prepared in which 1.1 g of purified glycomacropeptide (GMP) powder (available from Agropur, GMP 97.1% of protein, Moisture 6.9%, phenylalanine 1,454 ppm/whole solid content) was dissolved in 98 mL of ultrapure water, and the pH (25°C) was adjusted using a 1 M sulfuric acid aqueous solution. By changing addition amount of the 1 M sulfuric acid aqueous solution, four types of aqueous compositions (whey protein mixture) with a pH (25°C) of 4.2, 5.0, 6.0, and 6.5 were obtained. To each of the whey protein mixtures, 400 mg (dry weight basis) of the activated carbon A was added, and the mixture was stirred at room temperature of 25°C for 1 hour. Subsequently, the activated carbon suspensions were filtered through a filter, to obtain each clear GMP treated liquid. Each of the obtained treated liquids was dried under heating at 105°C, to obtain each GMP powder with a low phenylalanine content. In the results, when the sample with a pH (25°C) of 4.2 was used, the Phe concentration (in terms of solid content) in the obtained GMP powder was 635 ppm by mass. When the samples with pH (25°C) of 5.0, 6.0, and 6.5 were used, the Phe concentrations (solid content equivalent) in the obtained GMP powders were 805 ppm by mass, 1,027 ppm by mass, and 1,044 ppm by mass, respectively. Thus, it was confirmed that the GMP powders with the decreased Phe concentration were obtained at any pH (25°C).

The Phe concentration (in terms of solid content) in a control GMP powder that was obtained by the same manner as that described above except that activated carbon was not added was 1,454 ppm by mass.

### 2-2. Correlation between pH of whey protein mixture and Phe residual rate

FIG. 4 is a graph in which the pH of the whey protein mixture (horizontal axis) and the Phe residual rate (vertical axis) are plotted. The Phe residual rate was calculated by the same manner as that of 1-2 described above.

The isoelectric point of GMP is about 3.8. There is a tendency in which as the pH is closer to 3.8, the degree of hydrophobicity increases, and GMP is likely to adsorb to the activated carbon. In this respect, the Phe residual rate also decreases with a decrease in pH. Therefore, it seems that the Phe component removed by adsorption to the activated carbon is a compound similar to GMP.

### [Example 3]

### 3-1. Confirmation of presence or absence of Phe elution by washing activated carbon

It is considered that after a sample is treated with activated carbon, glycomacropeptide attached to the activated carbon is washed with water (to obtain glycomacropeptide with a low Phe content), to improve the yield of glycomacropeptide with a low Phe content. In this respect, the elution of the Phe-containing component that has adsorbed to the activated carbon is disadvantageous to accomplishment of an object for producing glycomacropeptide with a low Phe content. As a countermeasure to this, the Phe residual rate in the sample obtained by treating the sample with the activated carbon and then washing the activated carbon with water was confirmed, and an influence on the Phe residual rate of water-washing was confirmed.

### <Production Example 16 (No Washing)>

As a whey protein mixture, an aqueous composition was prepared in which 27.3 g of purified glycomacropeptide (GMP) powder (available from Agropur, GMP 97.1% of protein, Moisture 6.9%, phenylalanine 1,454 ppm/whole solid content) was dissolved in 473 mL of ultrapure water. To the whey protein mixture, 10.0 g (dry weight basis) of the activated carbon I was added, and the mixture was stirred at room temperature of 25°C for 1 hour. Subsequently, the activated carbon suspension was filtered through a filter, to obtain about 469 g of clear GMP treated liquid. The obtained treated liquid was dried under heating at 105°C, to obtain about 21.60 g (phenylalanine 713 ppm/whole solid content) of GMP powder with a low phenylalanine content.

### <Production Example 17>

A whey protein mixture was prepared by the same manner as that of Production Example 16. To the whey protein mixture, 10.0 g (dry weight basis) of the activated carbon I was added, and the mixture was stirred at room temperature of 25°C for 1 hour. Subsequently, the activated carbon suspension was filtered through a filter. Then, 10.5 g of ultrapure water was added to the activated carbon cake formed on the filter. Through these procedural steps, about 480 g of a clear GMP treated liquid containing ultrapure water with which the activated carbon cake was washed was obtained. The obtained treated liquid was dried under heating at 105°C, to obtain about 22.22 g (phenylalanine 704 ppm/whole solid content) of GMP powder with a low phenylalanine content.

### <Production Example 18>

By the same manner as that of Production Example 17 except that 30.1 g of ultrapure water was added to the activated carbon cake formed on the filter, about 502 g of a clear GMP treated liquid containing ultrapure water with which the activated carbon cake was washed was obtained. The obtained treated liquid was dried under heating at 105°C, to obtain about 22.43 g (phenylalanine 723 ppm/whole solid content) of GMP powder with a low phenylalanine content.

### <Production Example 19>

By the same manner as that of Production Example 17 except that 70.5 g of ultrapure water was added to the activated carbon cake formed on the filter, about 539 g of a clear GMP treated liquid containing ultrapure water with which the activated carbon cake was washed was obtained. The obtained treated liquid was dried under heating at 105°C, to obtain about 23.25 g (phenylalanine 708 ppm/whole solid content) of GMP powder with a low phenylalanine content.

### 3-2. Correlation between washing water proportion of activated carbon and Phe residual rate

The proportions of ultrapure water used in washing the activated carbon (washing water proportion) and the Phe residual rates in Production Examples 16 to 19 are shown in Table 4. The washing water proportion is the amount (% by mass) of ultrapure water used in washing relative to a dry weight (dry mass) of the added activated carbon of 100% by mass.

### [Table 4]

**(TABLE 4)**

| | | PRODUCTION EXAMPLE | | | |
|---|---|---|---|---|---|
| | | 16 | 17 | 18 | 19 |
| ACTIVATED CARBON | No. | I | I | I | I |
| | PORE VOLUME [mL/g] | 1.27 | 1.27 | 1.27 | 1.27 |
| | AVERAGE PORE DIAMETER [nm] | 3.60 | 3.60 | 3.60 | 3.60 |
| | SPECIFIC SURFACE AREA [m²/g] | 1414 | 1414 | 1414 | 1414 |
| AMOUNT OF ADDED ACTIVATED CARBON [%]^{∗} | | 40 | 40 | 40 | 40 |
| AMOUNT OF WASHING WATER [g] | | 0 | 10.5 | 30.1 | 70.5 |
| WASHING WATER PROPORTION [%]^{∗∗} | | 0 | 105 | 301 | 705 |
| C₁ [ppm]^{∗1} | | 713 | 704 | 723 | 708 |
| C₂ [ppm]^{∗2} | | 1454 | 1454 | 1454 | 1454 |
| Phe RESIDUAL RATE [%] | | 49.0 | 48.4 | 49.7 | 48.7 |

| | | | | | |
|---|---|---|---|---|---|
| ^{∗} VALUE (% BY MASS) RELATIVE TO WHOLE SOLID CONTENT OF 100% BY MASS IN WHEY PROTEIN MIXTURE ^{∗∗} VALUE (% BY MASS) RELATIVE TO DRY WEIGHT OF ADDED ACTIVATED CARBON OF 100% BY MASS ^{∗1} Phe CONCENTRATION IN SAMPLE AFTER TREATMENT WITH ACTIVATED CARBON ^{∗2} Phe CONCENTRATION IN CONTROL SAMPLE | | | | | |

According to the measurement results of the Phe residual rate in the GMP treated liquid obtained under conditions of washing water proportion of 0 to 705%, even when the washing water proportion increased, the Phe residual rate in the sample was not changed, as shown in Table 4. That is, even when the sample was treated with the activated carbon and the activated carbon was then washed with water, the Phe-containing component that had adsorbed to the activated carbon was not eluted. Thus, it was confirmed that it was possible to increase the yield of glycomacropeptide with a low Phe content by treating the sample with the activated carbon and then washing glycomacropeptide attached to the activated carbon with water.

## Claims

1. A method for producing glycomacropeptide, comprising:
(A) bringing a whey protein mixture containing glycomacropeptide into contact with activated carbon; and
(B) separating the glycomacropeptide from the activated carbon.

2. The method according to claim 1, wherein the whey protein mixture is crude glycomacropeptide derived from whey.

3. The method according to claim 1 or 2, wherein a pore volume of the activated carbon is 0.50 mL/g or more.

4. The method according to any one of claims 1 to 3, wherein the activated carbon is derived from the plant-based carbonaceous precursor.

5. The method according to any one of claims 1 to 4, wherein an average pore diameter of the activated carbon is 2 nm or more.

6. The method according to any one of claims 1 to 5, wherein a specific surface area of the activated carbon is 1,000 m²/g or more.

7. The method according to any one of claims 1 to 6, wherein the whey protein mixture contains a protein containing a phenylalanine residue.

8. The method according to any one of claims 1 to 7, wherein in step (B), separating glycomacropeptide with a phenylalanine concentration of 1,000 ppm by mass or less (in terms of solid content).
